# EUROPÄISCHE PATENTSCHRIFT

(11) **EP 3 202 346 B1**
(45) Veröffentlichungstag und Bekanntmachung des Hinweises auf die Patenterteilung: **24.11.2021**
(21) Anmeldenummer: 17153122.1
(22) Anmeldetag: 25.01.2017
(51) Int. Cl.: A61B 17/34

(54) **TROKARHÜLSE, TROKARSYSTEM SOWIE VERFAHREN ZUR HERSTELLUNG EINER TROKARHÜLSE**
TROCAR SLEEVE, TROCAR SYSTEM AND METHOD FOR PRODUCING A TROCAR SLEEVE
DOUILLE DE TROCART, SYSTÈME DE TROCART ET PROCÉDÉ DE FABRICATION D'UNE DOUILLE DE TROCART

(30) Priorität: 27.01.2016 DE 102016101462
(43) Veröffentlichungstag der Anmeldung: 09.08.2017
(73) Patentinhaber: Karl Storz SE & Co. KG, 78532 Tuttlingen (DE)
(72) Erfinder: Wagner, Sebastian, 75015 Bretten (DE); Sauer, Michael, 78532 Tuttlingen (DE); Fuchs, Alexander, 78256 Steißlingen (DE)
(74) Vertreter: Witte, Weller & Partner Patentanwälte mbB

(56) Entgegenhaltungen:
- EP-A1- 2 090 258
- WO-A1-2007/057127
- WO-A2-2013/135354
- DE-A1- 2 218 901
- DE-A1-102011 107 615
- US-A- 5 423 848

## Beschreibung

Die Erfindung betrifft ein Trokarsystem, mit einer Trokarhülse und einem Trokardorn, wobei die Trokarhülse mit einem flexiblen Hohlschaft, der ein distales Ende und ein proximales Ende aufweist, und mit einem Handhabungskopf versehen ist, der am proximalen Ende des Hohlschaftes ausgebildet ist, und wobei die Trokarhülse aus einem elastomeren Werkstoff hergestellt ist. Schließlich betrifft die Offenbarung ferner ein Verfahren zur Bereitstellung eines Trokarsystems, umfassend die Herstellung einer Trokarhülse.

Aus der DE 42 34 990 A1 ist eine flexible Trokarhülse bekannt, die ein Rohr und einen Aufsatz umfasst, wobei ein distaler Bereich des Rohres durch eine Trokareinstichstelle ins Körperinnere einführbar ist, wobei der Aufsatz an einem proximalen Ende des Rohres angebracht ist, wobei der Aufsatz an seiner proximalen Stirnseite eine Öffnung aufweist, der eine Dichtung zugeordnet ist, und wobei das Rohr aus einem flexiblen Material besteht. Zur Kopplung des Rohres und des Aufsatzes ist ein Zwischenrohr vorgesehen.

Aus der DE 43 12 147 A1 ist ein Trokar bekannt, mit einem Obturator, der eine Bohrspitze zur Durchbohrung von Körpergewebe aufweist, und einer Kanüle zum Ausbilden einer Passage in dem von der Bohrspitze durchbohrten Gewebe, so dass ein Endoskop oder ein chirurgisches Werkzeug in eine Körperhöhle einführbar ist, wobei die Kanüle aus einer flexiblen Röhre oder einer Schraubenfederwicklungs-Röhre geformt ist.

Aus der DE 101 56 312 A1 ist ein Schaft für einen chirurgisch hergestellten Zugang bekannt, der an seinem distalen Ende an seiner Außenfläche einen Gewindesteg aufweist, welcher sich in Umfangsrichtung des Schaftes nur über einen Teilbereich des Umfanges erstreckt.

Aus der EP 0 535 974 A1 ist ein Trokarsystem mit einem flexiblen Trokarrohr bekannt.

Trokarhülsen dienen allgemein dazu, bei einem minimalinvasiven Eingriff einen Zugang zu einer Körperhöhle bereitzustellen. Trokarsysteme umfassend Trokarhülsen sowie Trokardorne werden beispielsweise bei der Laparoskopie verwendet, um einen Zugang zur Bauchhöhle zu schaffen. Zu diesem Zweck umfasst die Trokarhülse einen Hohlschaft. Beim Setzen der Trokarhülse wird das Trokarsystem über eine Inzision in der Bauchdecke oder allgemein in einem Gewebe angesetzt und durch diese hindurchgeschoben. Danach ragt das distale Ende des Schaftes in die Bauchhöhle hinein, das proximale Ende steht von der Oberseite der Bauchdecke oder des Gewebes ab. Aus Sicht eines Bedieners oder Operateurs ist das distale Ende das dem Operateur abgewandte Ende. Das proximale Ende ist das dem Operateur zugewandte Ende.

Die Kombination aus Trokarhülse und Trokardorn wird allgemein als Trokar bzw. Trokarsystem bezeichnet. Ausführungsformen von Trokarsystemen umfassen eine Trokarhülse sowie einen Trokardorn, der in den Hohlschaft der Trokarhülse einschiebbar ist. Üblicherweise weist der Trokardorn an seinem distalen Ende eine Spitze auf. Die Spitze reicht über das distale Ende der Trokarhülse hinaus, wenn der Trokardorn vollständig in diese eingeschoben ist. Gemeinsam können die Trokarhülse und der Trokardorn durch die Bauchdecke oder ein anderes Gewebe hindurchgeschoben werden, um eine Zugangsöffnung zu schaffen. Nach dem Setzen der Trokarhülse wird der Trokardorn abgezogen bzw. ausgezogen. Somit können durch die verbliebene Ausnehmung im Hohlschaft Instrumente in die Körperhöhle oder Körperöffnung eingeführt werden.

Trokarhülsen können ferner ein proximales Kopfteil aufweisen, das auch im gesetzten Zustand der Trokarhülse üblicherweise nicht in das Körperinnere eindringt. Das Kopfteil kann auch als Handhabungskopf bezeichnet werden. Beispielhaft weist das Kopfteil Anschlüsse auf, um ein Insufflationsgas, Spülflüssigkeiten oder dergleichen durch die Trokarhülse hindurchzuführen. Darüber hinaus kann am Kopfteil selbst oder zumindest dem Kopfteil benachbart bei der Trokarhülse eine Dichtungsanordnung vorgesehen sein, um einen Verschluss bzw. eine Abdichtung des Trokarsystems zu ermöglichen.

Trokarhülsen selbst, insbesondere deren Hohlschäfte, können grundsätzlich aus einem hinreichend steifen und starren Werkstoff gefertigt sein, insbesondere aus Metallwerkstoffen. Es sind jedoch auch sogenannte flexible Trokarhülsen bzw. Trokarhülsen mit flexiblen Hohlschäften bekannt, die verformbar bzw. verbiegbar sind. Dies ist beispielsweise bei der Durchführung gebogener Instrumente bzw. auslenkbarer Instrumentenschäfte von Vorteil.

Derartige flexible Hohlschäfte können beispielhaft aus thermoplastischen oder duroplastischen Werkstoffen hergestellt werden. Üblicherweise ist eine derartige Trokarhülse als gebaute, mehrteilige Trokarhülse ausgeführt, wobei zumindest das Kopfteil und der Hohlschaft separate Komponenten sind, die miteinander gefügt werden.

Weitere beispielhafte Ausführungsformen von Trokarhülsen umfassen einen Gewindeabschnitt, der ausgehend vom proximalen Ende der Trokarhülse in Richtung auf das distale Ende versetzt ist. Mit anderen Worten kann ein derartiger Gewindeabschnitt, der etwa helixartig gestaltet ist, entlang der Längserstreckung des Hohlschaftes vorgesehen sein und sich von diesem ausgehend spiralförmig nach außen erheben. Eine Trokarhülse mit einer derartigen Gestaltung ist beispielsweise in der nachveröffentlichten EP 3 009 088 A1 beschrieben.

Beispielhaft ist die Helix an einem Längsabschnitt des Hohlschaftes ausgebildet, der vom Kopfteil derart beabstandet ist, dass die eingeführte Trokarhülse außerhalb des Körpers durch das Kopfteil sowie innerhalb des Körpers durch die Helix gestützt wird, wobei im gesetzten Zustand zwischen der Helix und dem Kopfteil eine Gewebeschicht (etwa die Bauchdecke) angeordnet ist. Somit kann die Helix eine innere Anlagefläche bereitstellen. Das Kopfteil kann eine äußere Anlagefläche bereitstellen. Es sind auch Ausgestaltungen vorstellbar, bei denen zwischen der Helix und dem Kopfteil ein weiterer Gewindeabschnitt (oder insgesamt ein durchgängiges Gewinde) ausgebildet ist, auf dem eine Art Kontermutter zur Fixierung der Trokarhülse zwischen der Helix und der Kontermutter sitzt.

In diesem Zusammenhang wird ferner auf die DE 101 56312 A1 verwiesen, die einen Schaft für einen chirurgisch hergestellten Zugang offenbart, wobei der Schaft an seinem distalen Ende an seiner Außenfläche einen Gewindesteg aufweist.

Bekannte Trokarhülsen mit zumindest hinreichend flexibel gestalteten Hohlschäften weisen üblicherweise einen mehrstückigen Aufbau auf, insbesondere betreffend die Verbindung zwischen dem Hohlschaft und dem Kopfteil. Es besteht häufig ein Widerspruch zwischen der gewünschten hohen Flexibilität und der gleichzeitig erforderlichen Stabilität des Hohlschaftes.

Ferner sind Trokarhülsen mit wellrohrartig gestalteten Hohlschäften bekannt. Diese Gestaltung (korrugierte Rohre) erhöht die Flexibilität des Hohlschaftes. Ähnliches kann etwa mit einem gewindeartigen Spiralprofil bewirkt werden, das außen am Hohlschaft eine Erhebung und innen am Hohlschaft eine entsprechende Vertiefung aufweist, wobei die Wandstärke des Hohlschaftes im Wesentlichen konstant bleiben kann. Mit anderen Worten erstreckt sich die Außenwand des Hohlschaftes mäanderförmig. Beide Maßnahmen haben den Nachteil, dass die Relativbewegung zwischen dem Instrument und dem Hohlschaft erschwert wird, da die Innenkontur des Hohlschaftes nicht glatt ist. Darüber hinaus neigen derartig gestaltete Hohlschäfte zum Knicken bzw. zur Faltenbildung.

Vor diesem Hintergrund liegt der Erfindung die Aufgabe zugrunde, ein Trokarsystem mit einer Trokarhülse anzugeben, die einerseits hochflexibel gestaltet ist und sich somit für gebogene Instrumente eignet. Ferner soll die Trokarhülse hinreichend fest und steif gestaltet sein, um das definierte Setzen des Trokars bzw. das Einführen der Trokarhülse zu vereinfachen. Darüber hinaus soll die Trokarhülse möglichst wirtschaftlich herstellbar sein. Insbesondere soll nach Möglichkeit auf zusätzliche Montageschritte bzw. Fertigungsschritte verzichtet werden können. Die Trokarhülse soll ferner nach Möglichkeit Zusatzfunktionen bereitstellen. Darüber hinaus soll die Trokarhülse eine hohe Biokompatibilität aufweisen. Strukturelle Schnittstellen zwischen beteiligten Komponenten sollen weitgehend vermieden werden. Schließlich soll im Rahmen der vorliegenden Offenbarung ein korrespondierendes Verfahren zur Bereitstellung eines Trokarsystems umfassend eine Trokarhülse sowie einen Trokardorn angegeben werden.

Das Trokarsystem betreffend wird die Aufgabe dadurch gelöst, dass der Hohlschaft eine Innenkontur aufweist, die derart an eine Außenkontur des Trokardorns angepasst ist, dass die Trokarhülse und der Trokardorn unter Erzeugung einer definierten Vorspannung miteinander koppelbar sind, um die Trokarhülse zu stabilisieren, dass der Trokardorn unter Erzeugung der definierten Vorspannung in die Trokarhülse einführbar ist, und dass zumindest der Hohlschaft und der Handhabungskopf einstückig gestaltet sind.

Erfindungsgemäß ist nämlich die Trokarhülse derart ausgeführt, dass sich im Zusammenspiel mit dem Trokardorn eine definierte, zumindest partielle Aufweitung (radiale Dehnung nach Außen) ergibt, die eine Vorspannung bewirkt. Die Vorspannung versteift die Trokarhülse bzw. erhöht die Festigkeit der Trokarhülse, insbesondere beim Hohlschaft. Auf diese Weise kann die Trokarhülse bzgl. ihrer Längsachse druckbelastet, zugbelastet bzw. torsionsbelastet werden, ohne dass es zu übermäßigen Verformungen, Stauchungen und dergleichen kommt. Nach dem Setzen der Trokarhülse kann der Trokardorn entfernt werden. Auf diese Weise wird die Aufweitung rückgängig gemacht und die Vorspannung abgebaut. Somit steht dann eine hohe Flexibilität/Biegsamkeit insbesondere des Hohlschaftes zur Verfügung.

Auf diese Weise kann die Trokarhülse einerseits hochflexibel gestaltet sein. Andererseits ist eine deutlich erhöhte Festigkeit gegeben, wenn der Trokardorn in definierter Weise in den Hohlschaft eingebracht ist. Die Trokarhülse kann definiert geführt werden.

Mit anderen Worten ist die Innenkontur des Hohlschaftes bewusst etwas zu klein für eine gegebene Außenkontur des Trokardorns gestaltet. Auf diese Weise wird die Innenkontur und somit der gesamte Hohlschaft auf das durch die Außenkontur des Trokardorns vorgegebenen Nennmaß aufgeweitet. Mit anderen Worten weist die Innenkontur im unbelasteten Zustand eine definierte Überdeckung in Bezug auf die Außenkontur des Hohlschaftes auf.

Gemäß dem obigen Aspekt kann die Trokarhülse ohne separate Stützelemente ausgeführt sein und allein durch das Einbringen des Trokardorns eine hohe Stabilität aufweisen. Dies ist eine Voraussetzung für die möglichst integrale Gestaltung der Trokarhülse. Die Trokarhülse weist eine hohe Eigenstabilität auf, wenn der flexible Hohlschaft durch den Trokardorn zumindest abschnittsweise aufgeweitet ist.

Die Innenkontur des Hohlschaftes kann beispielsweise als Durchgangsloch oder Durchgangskanal ausgeführt sein. Die Innenkontur kann grundsätzlich einen runden Querschnitt umfassen. Andere Querschnittsformen sind vorstellbar. Der Trokardorn weist üblicherweise eine Außenkontur auf, die an die Innenkontur des Hohlschaftes angepasst ist. Demgemäß kann auch der Trokardorn eine Außenkontur mit runden oder kreisförmigen Querschnitten umfassen. Es ist jedoch auch vorstellbar, den Hohlschaft und den Trokardorn derart zu gestalten, dass sich bei der Innenkontur und bei der Außenkontur nicht korrespondierende bzw. nicht kongruente Querschnitte ergeben. Beispielhaft kann der Trokardorn zumindest abschnittsweise einen ovalen Querschnitt aufweisen, wogegen die Innenkontur des Hohlschaftes einen kreisförmigen Querschnitt aufweist oder zumindest einen ovalen Querschnitt mit einem Seitenverhältnis, das vom Seitenverhältnis des Querschnitts der Außenkontur abweicht.

Es versteht sich, dass der Hohlschaft nicht vollständig entlang seiner Längserstreckung durch den Trokardorn aufgeweitet werden muss. Vielmehr ist es vorstellbar, dass der Hohlschaft abschnittsweise entlang seiner Längserstreckung und/oder abschnittsweise entlang seiner Umfangserstreckung durch den Trokardorn aufgeweitet wird.

Der flexible Hohlschaft ist zur Aufnahme eines Trokardorns und zur Durchführung von Instrumenten ausgebildet. Durch ein definiertes Einführen des Trokardorns in den Hohlschaft kann die Überdeckung bzw. Aufweitung beeinflusst werden. Beispielsweise kann die Aufweitung der Innenkontur des Hohlschaftes von einer axialen Relativlage zwischen dem Trokardorn und dem Hohlschaft abhängig sein. Dies gilt insbesondere dann, wenn zumindest die Innenkontur oder die Außenkontur zumindest abschnittsweise konisch oder sphärisch gestaltet sind.

In einer Ausgestaltung der Trokarhülse ist der Hohlschaft in einer definierten axialen Relativlage zwischen den Trokarhülse und dem Trokardorn zumindest abschnittsweise aufgeweitet. Dies betrifft einen Zustand, in dem der Trokardorn in die Trokarhülse eingefügt ist. Auf diese Weise kann ein Verbund geschaffen werden, um die Trokarhülse durch die Bauchdecke oder das Gewebe zumindest abschnittsweise in das Körperinnere einzuführen.

Bei dem elastomeren Werkstoff handelt es sich insbesondere um einen Werkstoff, der eine Shore Härte von höchstens 90 Shore A aufweist. Bei Elastomeren handelt es sich allgemein um Werkstoffe, die hinreichend formfest gestaltet sind aber gleichwohl elastisch verformbar sind. Üblicherweise werden Elastomere in einem Temperaturbereich eingesetzt, der über der Glasübergangstemperatur bzw. über dem Glasübergangspunkt liegt. Elastomere Werkstoffe zeigen allgemein ein gummielastisches Verhalten.

Beispielhaft kann zumindest der Hohlschaft, insbesondere dessen Innenkontur, oder der Trokardorn, insbesondere dessen Außenkontur, zumindest abschnittsweise konisch gestaltet sein. Mit anderen Worten kann sich zumindest der Querschnitt der Innenkontur oder der Querschnitt der Außenkontur vom proximalen Ende in Richtung auf das distale Ende hin verjüngen. Demgemäß kann über einen gewählten axialen Versatz zwischen dem Trokardorn und der Trokarhülse der Grad der Überdeckung bzw. der Aufweitung bestimmt werden. Somit kann auch die Höhe der Vorspannung im Hohlschaft der Trokarhülse beeinflusst werden. Je höher die Vorspannung des Hohlschaftes, desto höher ist die Eigenstabilität beim Einführen in das Körperinnere.

Es ist vorstellbar, eine einzige definierte axiale Relativlage vorzusehen, welche im Wesentlichen zu genau einer definierten Überdeckung und somit zu einer definierten Vorspannung führt. Dies kann etwa durch geeignete Anschläge bewerkstelligt werden, um das axiale Eindringen bzw. Durchdringen des Trokardorns in Bezug auf die Trokarhülse zu begrenzen. Umgekehrt ist es vorstellbar, bewusst keinen Anschlag vorzusehen, um eine Variation der axialen Relativlage (des axialen Versatzes) und somit der Vorspannung zu erlauben.

Gemäß einer weiteren Ausgestaltung beträgt die Shore Härte des elastomeren Werkstoffs, bestimmt gemäß DIN ISO 7619-1, maximal 90 Shore A, vorzugsweise maximal 70 Shore A. Ein minimaler Wert für die Härte kann etwa 50 Shore A betragen. Denkbare Bereiche für die Shore Härte des elastomeren Werkstoffs liegen zumindest gemäß einigen Ausführungsbeispielen zwischen 50 und 90 Shore A, vorzugsweise zwischen 50 und 70 Shore A. Je niedriger der Wert der Shore Härte, desto schwächer ist die Vernetzung des Werkstoffs und desto weicher ist das Material. Gemäß einer beispielshaften Ausführungsform ist eine Shore Härte von etwa 70 Shore A (bzw. von 70 Shore A) vorgesehen. Es versteht sich, dass übliche Toleranzen, mit denen bei der Gestaltung und Verarbeitung elastomerer Werkstoffe zu rechnen ist, hiervon umfasst sind.

Zumindest der Hohlschaft und der Handhabungskopf der Trokarhülse sind einstückig gestaltet, wobei die Trokarhülse vorzugsweise integral gestaltet und urformend hergestellt ist. Auf diese Weise kann der Herstellungsaufwand und insbesondere der Montageaufwand deutlich reduziert werden. Vorzugsweise ist die Trokarhülse vollständig oder nahezu vollständig integral gefertigt. Insbesondere der Hohlschaft und der Handhabungskopf sind vorzugsweise einstückig geformt und gemeinsam hergestellt. Es versteht sich, dass insbesondere der Handhabungskopf mit weiteren Elementen bestückt werden kann, etwa mit Dichtungsanordnungen, Anschlüssen, Ventilen und dergleichen.

Gleichwohl führt die gemeinsame, integrale Gestaltung des Hohlschaftes und des Handhabungskopfes aus dem elastomeren Werkstoff zu einer fertigungstechnischen Optimierung. Es gibt keine strukturelle Schnittstelle und keine Schnittstelle zwischen verschiedenen Werkstoffen beim Übergang zwischen dem Hohlschaft und dem Handhabungskopf. Dies ist hygienisch vorteilhaft.

Gemäß einer weiteren Ausgestaltung der Trokarhülse ist der elastomere Werkstoff aus der Gruppe ausgewählt, die aus Folgenden besteht: Silikone, Silikonkautschuk, thermoplastische Elastomere (TPE), Kautschuk sowie diese enthaltende Compounds. Kautschuk umfasst synthetischen Kautschuk und Naturkautschuk. Bei dem Elastomer handelt es sich zum Beispiel um Ethylen-Propylen-Dien-Kautschuk (EPDM).

Gemäß einer weiteren Ausgestaltung der Trokarhülse ist der Hohlschaft zumindest abschnittsweise rohrartig oder zylinderartig gestaltet und weist zumindest abschnittsweise eine glatte erhebungsfreie Außenfläche auf. Vorzugsweise ist die Innenkontur zumindest abschnittsweise mit einer reibungsmindernden Beschichtung versehen. Die reibungsmindernde Beschichtung kann beispielsweise aus Polytetrafluorethylen (PTFE) bestehen oder dieses umfassen. Andere Stoffe zur Reibungsminimierung bzw. Reibungsoptimierung sind denkbar.

Zur Minimierung der Reibung zwischen dem Hohlschaft und einzuführenden Instrumenten bzw. dem Trokardorn kann eine Fluorierung zumindest eines Kontaktbereichs der Innenkontur des Hohlschaftes vorgesehen sein.

Die Innenkontur des Hohlschaftes ist vorzugsweise zumindest in dem Bereich, in dem dieser durch den Trokardorn kontaktiert wird, glatt ausgeführt. Mit anderen Worten gibt es kein sich axial erstreckendes Wellprofil mit umlaufenden Abschnitten bzw. keine schraubenförmigen Furchen bei der Innenkontur. Dies vereinfacht die Handhabung und Durchführung und Instrumenten durch den Hohlschaft. Insbesondere das Anhaften bzw. Klemmen von Instrumenten wird deutlich erschwert.

Vorzugsweise ist auch die Außenkontur oder Außenfläche des Hohlschaftes zumindest abschnittsweise glatt und ohne Erhebungen ausgeführt. Gleichwohl versteht sich, dass zumindest ein Gewindeabschnitt an der Außenkontur des Hohlschaftes ausgebildet sein kann. Jedoch ist der Hohlschaft der Trokarhülse insbesondere zur Erhöhung der Biegsamkeit bzw. Flexibilität nicht mit einer wellrohrartigen Gestaltung versehen. Mit anderen Worten ist der Hohlschaft vorzugsweise nicht-korrugiert ausgeführt.

Gemäß einer weiteren Ausgestaltung der Trokarhülse weist der Hohlschaft zumindest abschnittsweise eine sich von einem distalen Ende hin zu einem proximalen Ende erhöhende Wandstärke auf. Insbesondere kann es sich um eine stetige oder quasi-stetige Erhöhung der Wandstärke handeln. Diese Maßnahme hat den Vorteil, dass der Hohlschaft selbst in einem Übergangsbereich mit dem Handhabungskopf hinreichend fest gestaltet ist. Das distale Ende des Hohlschaftes, das nur relativ geringe Wandstärken aufweist, kann demgegenüber durch den einzuführenden Trokardorn einfach aufgeweitet werden, wobei eine Vorspannung erzeugt wird, die die Festigkeit der Trokarhülse erhöht.

Gemäß einer weiteren Ausgestaltung der Trokarhülse ist die Innenkontur des Hohlschaftes zumindest abschnittsweise konisch oder sphärisch mit einem von einem distalen Ende hin zu einem proximalen Ende größer werdenden Innendurchmesser gestaltet. Auch auf diese Weise kann das distale Ende des Hohlschaftes besonders einfach aufgeweitet werden, wodurch sich die Stabilität des Hohlschaftes im Verbund mit dem Trokardorn erhöht. Ferner kann der Hohlschaft insgesamt in Richtung auf das distale Ende verjüngt sein, was das Eindringen des Trokarsystems weiter vereinfacht. Gemäß einer Weiterbildung der obigen Ausgestaltung ist die Innenkontur des Hohlschaftes an eine zumindest abschnittweise konische oder sphärische Gestaltung der Außenkontur des Trokardorns angepasst, um die Trokarhülse beim Einführen des Trokardorns definiert vorzuspannen. Auch dies kann zur gewünschten Festigkeitssteigerung bzw. zur Erhöhung der Stabilität der Trokarhülse beitragen.

Gemäß einer weiteren Ausgestaltung der Trokarhülse ist am Hohlschaft ein Gewindeabschnitt ausgebildet, wobei der Hohlschaft, der Gewindeabschnitt und der Handhabungskopf einstückig gestaltet sind. Vorzugsweise ist der Gewindeabschnitt als Erhebung an einem Außenumfang des Hohlschaftes ausgeformt. Gemeinsam können der Hohlschaft, der Gewindeabschnitt und der Handhabungskopf aus demselben elastomeren Werkstoff gefertigt sein.

Es sind Ausgestaltungen vorstellbar, bei denen ein erster und ein zweiter Gewindeabschnitt am Hohlschaft ausgebildet ist, die axial voneinander versetzt sind. Der zweite Gewindeabschnitt kann genutzt werden, um eine Kontermutter aufzunehmen, die zwischen dem Handhabungskopf und dem ersten Gewindeabschnitt am Hohlschaft aufnehmbar ist. In diesem Zusammenhang wird erneut auf die nachveröffentlichte EP 3 009 088 A1 verwiesen.

Vorteilhaft ist ferner, dass ein Fertigungsverfahren gewählt werden kann, das auch die Entformung/Ausformung zumindest teilweise hinterschnittiger Gestaltungen erlaubt. Insofern ist eine große gestalterische Freiheit gegeben.

Der Gewindeabschnitt kann zumindest einen Gewindegang umfassen, dessen Höhe und Steigung variabel gestaltbar ist. Ferner kann etwa die Neigung von Flanken des Gewindeganges angewählte Einsatzbedingungen angepasst werden. Insgesamt kann ein im Wesentlichen frei definierbares Gewinde erzeugt werden, das einerseits ein leichtes Eindringen erlaubt, etwa durch eine hohe Steigung sowie eine geringe Höhe am distalen Auslauf des Gewindes. Ferner kann das Gewinde an seinem proximalen Auslauf etwa eine deutlich verringerte Steigung und eine größere Höhe als am distalen Auslauf aufweisen. Dies erhöht die Haltewirkung, insbesondere gegen versehentliches Herausziehen oder Herausreißen des Hohlschaftes aus dem Gewebe.

Gemäß einer weiteren Ausgestaltung der Trokarhülse ist die Innenkontur des Hohlschaftes zumindest abschnittsweise nicht-rotationsymmetrisch ausgeführt. Insbesondere kann die Innenkontur einen unrunden Querschnitt senkrecht zur Längsrichtung bzw. einer damit korrespondierenden Längsachse des Hohlschaftes aufweisen.

Auf diese Weise kann zwischen dem Hohlschaft der Trokarhülse und dem Trokardorn eine formschlüssige Drehmitnahmesicherung bewirkt werden. Demgemäß ist es von Vorteil, wenn auch der Trokardorn eine korrespondierende nichtrotationssymmetrische Gestaltung aufweist.

Beispielhaft weist der Trokardorn zumindest abschnittsweise entlang seiner Längserstreckung zumindest eine wulstartige Erhebung auf, die an eine wulstartige Vertiefung des Hohlschaftes angepasst ist, die sich auch in der Längsrichtung erstreckt. Demgemäß kann eine Relativverdrehung zwischen dem Hohlschaft und dem Trokardorn unterbunden bzw. zumindest reduziert werden.

In diesem Zusammenhang ist anzumerken, dass es gemäß zumindest einigen Ausgestaltungen nicht unbedingt darum geht, die Relativverdrehung zwischen dem Trokardorn und dem Hohlschaft vollständig zu unterbinden. Beispielhaft ist vorstellbar, dass der Hohlschaft bei einer Verdrehung des Trokardorns um dessen Längsachse zumindest abschnittsweise tordiert wird. Dies kann etwa dann der Fall sein, wenn zumindest abschnittsweise eine Drehmitnahme des Hohlschaftes durch den Trokardorn erfolgt, etwa aufgrund von Reibung zwischen der Innenkontur und der Außenkontur. Auch die Torsion oder Verdrillung des Hohlschaftes kann in gewünschter Weise eine Vorspannung beim Hohlschaft bewirken, die sich in einer erhöhten Stabilität niederschlägt.

Beispielhaft ist es vorstellbar, die Innenkontur des Hohlschaftes und die Außenkontur des Trokardorns mit einem ovalen Querschnitt oder einem ähnlichen nichtzylindrischen Querschnitt zu versehen. Auf diese Weise kann einerseits eine übermäßige Relativverdrehung zwischen dem Trokardorn und dem Hohlschaft vermieden werden. Jedoch kann eben zumindest in Grenzen eine Relativbewegung mit einer teilweisen Verdrillung des Hohlschaftes bewirkt werden, um die Festigkeit des Hohlschaftes zu steigern.

Gemäß einer weiteren Ausgestaltung der Trokarhülse ist am proximalen Ende des Hohlschaftes, insbesondere beim Handhabungskopf, eine Dichtungskontur oder eine Dichtungsaufnahmekontur ausgebildet. Mit anderen Worten sind Ausgestaltungen vorstellbar, bei denen auch eine Elastomerdichtung als integraler Bestandteil der Trokarhülse vorgesehen ist. Vorzugsweise ist die Dichtungskontur am Handhabungskopf aufgenommen. In alternativer Ausgestaltung ist am Handhabungskopf zumindest eine Dichtungsaufnahmekontur, etwa in Form einer umlaufenden Nut, zur Aufnahme einer separaten Dichtung vorgesehen.

Ein weiterer offenbarungsgemäßer Aspekt betrifft ein Trokarsystem, das eine Trokarhülse gemäß einem der hier beschriebenen Ausführungsbeispiele sowie einen Trokardorn umfasst, wobei der Trokardorn unter Erzeugung einer definierten Vorspannung in die Trokarhülse einführbar ist. Der Trokardorn wird in den Hohlschaft der Trokarhülse eingeführt und kann diesen zumindest abschnittsweise aufweiten. Auf diese Weise wird der Hohlschaft und somit die Trokarhülse stabilisiert. Vorzugsweise ist die erzielbare Vorspannung von einer axialen Relativlage zwischen dem Trokardorn und dem Hohlschaft abhängig.

Üblicherweise umfasst der Trokardorn an seinem distalen Ende eine Spitze. An die Spitze schließt sich ein Schaft an. Vorzugsweise ist der Schaft des Trokardorns zumindest abschnittsweise konisch oder sphärisch gestaltet. Insbesondere kann sich eine kegelartige Kontur ergeben. Auf diese Weise kann der Hohlschaft umso mehr aufgeweitet werden, je weiter der Trokardorn in den Hohlschaft eingeführt wird.

Die Aufgabe der Erfindung wird ferner durch ein Verfahren zur Bereitstellung eines Trokarsystems gelöst, das die folgenden Schritte umfasst:
- Herstellung einer Trokarhülse, vorzugsweise einer Trokarhülse nach einem der vorstehend genannten Aspekte, wobei das Verfahren die folgenden Schritte aufweist:
   - Bereitstellung einer Form, die zur Erzeugung einer Trokarhülse mit einem flexiblen Hohlschaft und einem Handhabungskopf ausgebildet ist,
   - Bereitstellung eines elastomeren Werkstoffs, vorzugsweise eines Werkstoffs der im verarbeiteten Zustand einer Shore Härte, bestimmt gemäß DIN ISO 7619-1, von maximal 90 Shore A, vorzugsweise maximal 70 Shore A, weiter bevorzugt maximal 50 Shore A aufweist, und
   - Urformen der Trokarhülse, umfassend Befüllen der Form, dabei einstückiges Formen des Hohlschaftes und des Handhabungskopfes, wobei am Hohlschaft eine Innenkontur ausgebildet wird, die derart an eine Außenkontur eines Trokardorns angepasst ist, dass die Trokarhülse und der Trokardorn unter Erzeugung einer definierten Vorspannung miteinander koppelbar sind, um die Trokarhülse zu stabilisieren,
- Bereitstellung eines Trokardorns, der an die Trokarhülse angepasst ist, und
- Einführen des Trokardorns in die Trokarhülse, dabei zumindest abschnittsweises Aufweiten der Trokarhülse, umfassend Erzeugung einer Vorspannung im Hohlschaft der Trokarhülse, zur mechanischen Stabilisierung der Trokarhülse.

Es versteht sich, dass die vorstehend genannten und die nachstehend noch zu erläuternden Merkmale der Erfindung nicht nur in der jeweils angegebenen Kombination, sondern auch in anderen Kombinationen oder in Alleinstellung verwendbar sind, ohne den Rahmen der vorliegenden Erfindung zu verlassen.

Weitere Merkmale und Vorteile der Erfindung ergeben sich aus der nachfolgenden Beschreibung mehrerer bevorzugter Ausführungsbeispiele unter Bezugnahme auf die Zeichnungen. Es zeigen:
- Fig. 1: eine perspektivische Darstellung eines Trokarsystems;
- Fig. 2: eine perspektivische Ansicht einer Trokarhülse, vom distalen Ende her;
- Fig. 3: einen gebrochenen Längsschnitt durch die Trokarhülse gemäß Fig. 2;
- Fig. 4: einen Längsschnitt durch ein Trokarsystem umfassend eine Trokarhülse gemäß den Fig. 2 und 3 sowie einen Trokardorn, in einer ersten axialen Relativlage;
- Fig. 5: das Trokarsystem gemäß Fig. 4 in einer zweiten axialen Relativlage;
- Fig. 6: eine vergrößerte Teilansicht eines distalen Endes eines Trokarsystems;
- Fig. 7: einen Längsschnitt durch eine weitere Ausführungsform einer Trokarhülse;
- Fig. 8: eine perspektivische Darstellung einer weiteren Trokarhülse, vom distalen Ende her;
- Fig. 9: einen Längsschnitt durch die Trokarhülse gemäß Fig. 8;
- Fig. 10: einen frontalen Schnitt durch die Trokarhülse gemäß Fig. 9, wobei die Ansichtsorientierung in Fig. 9 durch eine Linie X-X angedeutet ist;
- Fig. 11: einen frontalen Schnitt durch ein Trokarsystem umfassend eine Trokarhülse sowie einen Trokardorn, wobei die Ansichtsorientierung in Fig. 9 durch eine mit XI-XI bezeichnete Linie veranschaulicht wird;
- Fig. 12: eine perspektivische Ansicht einer weiteren Ausgestaltung einer Trokarhülse, vom distalen Ende her;
- Fig. 13: einen frontalen Schnitt durch die Trokarhülse gemäß Fig. 12 entlang der Linie XIII-XIII in Fig. 12;
- Fig. 14: ein schematisches Blockdiagramm zur Veranschaulichung eines Verfahrens zur Herstellung einer Trokarhülse; und
- Fig. 15: ein schematisches Blockdiagramm zur Veranschaulichung eines Verfahrens zur Bereitstellung eines Trokarsystems.

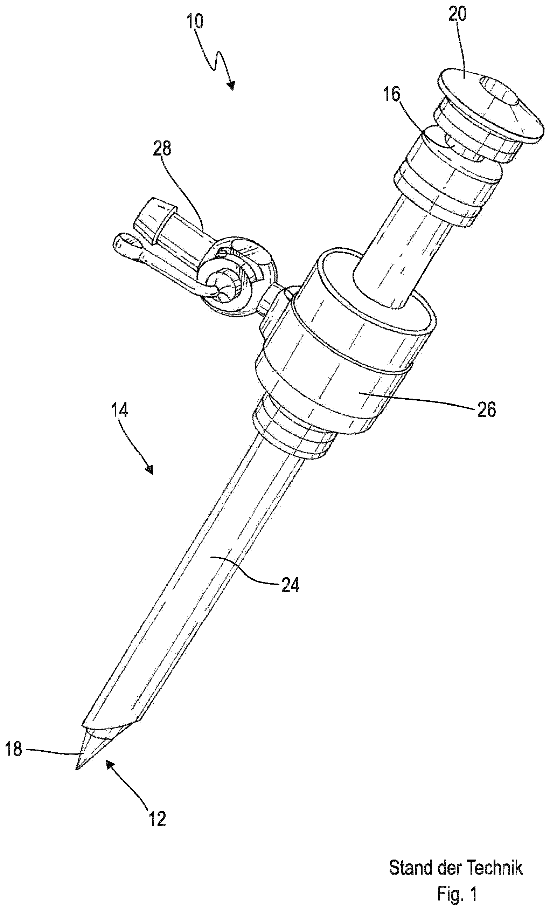

Mit Bezugnahme auf Fig. 1 wird eine bekannte Ausgestaltung eines Trokarsystems 10 veranschaulicht. Das Trokarsystem 10 wird allgemein auch als Trokar bezeichnet. Das Trokarsystem 10 umfasst einen Trokardorn 12 sowie eine Trokarhülse 14, in die der Trokardorn 12 einführbar ist, um das Trokarsystem 10 in eine Körperöffnung, etwa im Bauchraum, einzuführen. Das Trokarsystem 10 weist eine längliche Gestaltung auf, wobei der Trokardorn 12 und die Trokarhülse 14 eine gemeinsame Längsachse aufweisen.

Der Trokardorn 12 weist einen Schaft 16 auf, an dessen distalem Ende eine Spitze 18 ausgebildet ist. Am proximalen Ende des Schaftes 16 des Trokardorns 12 ist ein Handhabungsabschnitt 20 ausgebildet.

Die Trokarhülse 14 umfasst einen Hohlschaft 24, der auch als Tubus bezeichnet werden kann. Ferner umfasst die Trokarhülse 14 einen Handhabungskopf 26, der an einem proximalen Ende der Trokarhülse 14 oder dem proximalen Ende zumindest benachbart angeordnet ist. Beispielhaft weist der Handhabungskopf 26 einen mit einem Ventil versehenen Anschluss 28 auf, über den Spülflüssigkeiten, Gase oder dergleichen definiert zugeführt und abgeführt werden können.

Das in Fig. 1 veranschaulichte Trokarsystem 10 weist eine starre Trokarhülse 14 auf, insbesondere eine Trokarhülse 14 mit einem starren Hohlschaft 24. Es sind auch Trokarsysteme bekannt, die Trokarhülsen umfassen, die zumindest abschnittsweise flexibel und biegsam gestaltet sind. Dies ist insbesondere für die Durchführung gebogener Instrumente erforderlich.

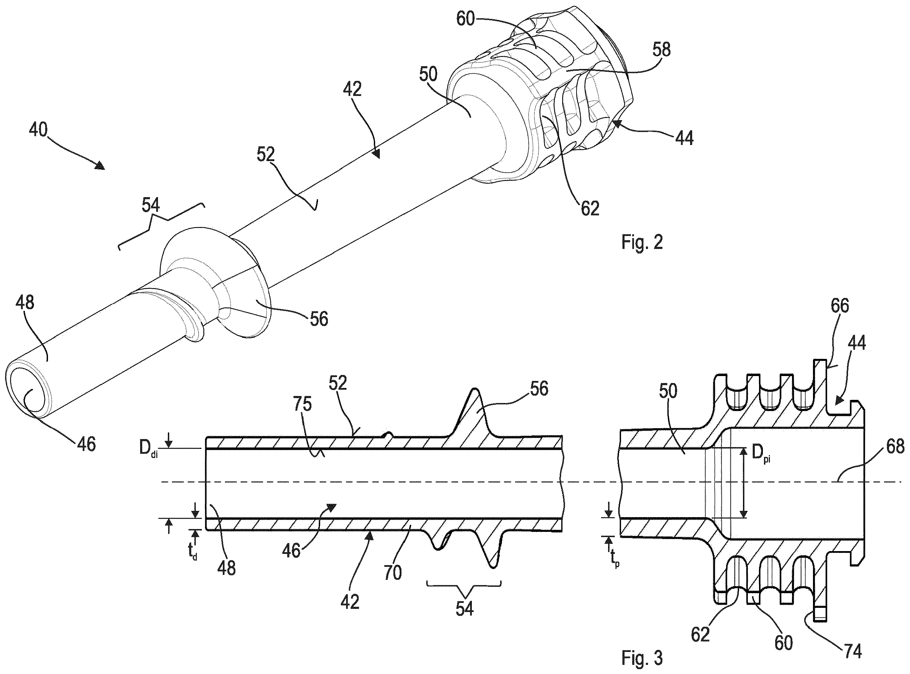

Mit Bezugnahme auf die Fig. 2 bis 13 werden verschiedene beispielhafte Ausgestaltungen von Trokarsystemen und insbesondere von Trokarhülsen veranschaulicht, deren Hohlschäfte flexibel und auslenkbar gestaltet sind. Insbesondere sind die nachfolgend veranschaulichten Trokarhülsen aus einem elastomeren Werkstoff mit einer definierten maximalen Shore Härte gefertigt. Ferner sind die Trokarhülsen einstückig gefertigt und integrieren verschiedene Komponenten, ohne dass es eines mehrstückigen Aufbaus bedarf.

Die nachfolgenden näher veranschaulichten Ausführungsbeispiele betreffen verschiedene Aspekte der Gestaltung und Herstellung von Trokarsystemen. Es versteht sich, dass einzelne Merkmale, die bei einer der Ausführungsformen vorgesehen sind, auch auf andere Ausführungsformen übertragbar sind, und zwar losgelöst von sonstigen Merkmalen der jeweiligen beispielhaften Ausführungsform.

Mit Bezugnahme auf die Fig. 2 und 3 wird eine erste Ausführungsform einer Trokarhülse näher veranschaulicht, die insgesamt mit 40 bezeichnet ist. Ähnlich wie in Fig. 1 gezeigt, kann die Trokarhülse 40 mit einem Trokardorn kombiniert werden, um ein Trokarsystem zu bilden.

Die Trokarhülse 40 umfasst einen Hohlschaft 42 und einen Handhabungskopf 44. Der Hohlschaft 42 und der Handhabungskopf 44 sind integral gestaltet und einstückig gefertigt. Gemeinsam definieren der Handhabungskopf 44 und der Hohlschaft 42 eine Längserstreckung der Trokarhülse 40, vergleiche auch eine in Fig. 3 durch 68 angedeutete Längsachse. Der Hohlschaft 42 definiert einen sich in Längsrichtung erstreckenden Durchlass 46, der zur Aufnahme eines Trokardorns oder eines Instruments dient.

Ferner umfasst der Hohlschaft 42 ein distales Ende 48 und ein proximales Ende 50. Das distale Ende 48 ist das Ende, das dem Inneren des Patienten zugewandt ist, wenn die Trokarhülse 40 an einem Gewebeabschnitt, insbesondere an einer Bauchdecke, des Patienten aufgenommen ist. Das proximale Ende 50 ist in diesem Zustand einem Bediener oder Operateur zugewandt und demgemäß vom Inneren des Patienten abgewandt. Am proximalen Ende 50 des Hohlschaftes 42 ist der Handhabungskopf 44 ausgebildet.

Der Hohlschaft 42 weist zumindest abschnittsweise eine glatte Oberfläche 52 auf, die insbesondere nicht als korrugierte Oberfläche bzw. gewellte Oberfläche gestaltet ist. Unbeschadet dessen ist gemäß zumindest einigen Ausgestaltungen am Hohlschaft 42 ein Gewindeabschnitt 54 ausgebildet, der etwa zumindest einen Gewindegang 56 umfasst, der auch als Helix bezeichnet werden kann. Vorzugsweise ist auch der Gewindeabschnitt 54 als integraler Bestandteil der Trokarhülse 40 gestaltet. In diesem Zusammenhang wird auf die gebrochene Schnittdarstellung gemäß Fig. 3 verwiesen. Es wird ersichtlich, dass der Hohlschaft 42, der Handhabungskopf 44 und, sofern vorhanden, der Gewindeabschnitt 54 einstückig gestaltet sind.

Die Steigung und die Höhe des Gewindegangs 56 können variabel sein und insbesondere spezifische Einsatzbedingungen berücksichtigen. Dies kann insbesondere eine große Steigung an einem distalen Auslauf des Gewindegangs 56 umfassen. An einem proximalen Auslauf des Gewindegangs 56 kann hingegen eine deutlich verringerte Steigung vorgesehen sein. Gleichermaßen kann der Gewindegang 56 an seinem distalen Ende zunächst eine sehr geringe Höhe aufweisen, die sich allmählich hin zu einer maximalen Höhe entwickelt, wobei hin zum proximalen Ende des Gewindegangs 56 eine stärkere Abnahme der Höhe (pro Umfangsabschnitt) vorgesehen ist. Diese Gestaltung hat den Vorteil, dass die Trokarhülse 40 einerseits leicht in Gewebe eindringen kann, da das distale Ende des Gewindegangs 56 gewissermaßen als Einführhilfe gestaltet ist. Hin zum proximalen Ende des Gewindegangs 56 sinkt die Steigung des Gewindegangs, ferner ist noch eine beträchtliche Höhe vorhanden. Auf diese Weise ist der mit dem Gewindeabschnitt 54 versehene Hohlschaft 42 gut gegen eine Schubbewegung in Richtung auf das proximale Ende gesichert. Es versteht sich, dass die hier erläuterte Ausgestaltung des Gewindeabschnitts 54 beispielhafter Natur ist.

Gleichwohl ist es vorteilhaft, die Trokarhülse 40 einstückig und urformend aus einem flexiblen Material, insbesondere einem elastomeren Material herzustellen. Dies erlaubt eine große gestalterische Freiheit und insbesondere das Anformen/Ausformen zusätzlicher Komponenten. Ein Vorteil kann darin gesehen werden, dass bei den gewählten Werkstoffen nicht mit einer hohen Wahrscheinlichkeit einer Lunkerbildung und dergleichen gerechnet werden muss. Somit muss nichts stets darauf geachtet werden, eine möglichst konstante Wandstärke bereitzustellen. Dies gilt insbesondere auch für den Gewindeabschnitt 54. Demgemäß kann sowohl die Oberfläche 54 an der Außenseite des Hohlschaftes 42 als auch eine Innenkontur 72 (vergleiche Fig. 3) an der Innenseite des Hohlschaftes 52 glatt gestaltet sein.

Einer Zusammenschau der Fig. 2 und 3 ist entnehmbar, dass der Handhabungskopf 44 gleichwohl eine Rippenstruktur aufweisen kann, um einerseits eine hohe Stabilität zu gewährleisten und andererseits Materialanhäufungen zu vermeiden.

Der Handhabungskopf 44 ist gegenüber dem Hohlschaft 42 radial vergrößert und stellt auch eine vergrößerte Aufnahmeöffnung bereit. Ferner ist der Handhabungskopf 44 mit sich im Wesentlichen axial erstreckenden Rippen 58 sowie mit sich im Wesentlichen umfänglich erstreckenden Rippen 60 versehen. Die Axialrippen 58 und die Umfangsrippen 60 bilden eine Kreuzstruktur. Die Rippen 58, 60 definieren bzw. umgeben Ausnehmungen oder Vertiefungen 62, die zur Vermeidung von Materialanhäufungen bzw. zur Gewichtsreduktion und zur Optimierung der Gewichtsverteilung beitragen. Der Hohlschaft 42 umfasst eine zylindrische Wandung 70, die die Längsachse 68 umgibt. An der Innenseite der Wandung 70 ist die Innenkontur 72 ausgebildet.

Ferner weist die anhand der Fig. 3 veranschaulichte Ausgestaltung der Trokarhülse 40 beim Handhabungskopf 44 eine nutförmige Aufnahme 74 auf, die etwa zur Aufnahme einer Dichtung dienen kann. Der Handhabungskopf 44 mündet in eine proximale Abschlussfläche 66, der jedoch die Aufnahme 74 vorgelagert sein kann.

Mit Bezugnahme auf den in Fig. 3 gezeigten Längsschnitt werden charakteristische Abmessungen der Trokarhülse 40 veranschaulicht. Ein distaler Innendurchmesser des Hohlschaftes 42 ist mit D_{di} bezeichnet. Ein proximaler Innendurchmesser des Hohlschaftes 42 ist mit Dₚᵢ bezeichnet. Eine Dicke oder Stärke der Wandung 70 beim distalen Ende 48 ist mit t_{d} bezeichnet. Eine Dicke der Wandung 70 beim proximalen Ende des Hohlschaftes 42 ist mit tₚ bezeichnet.

Es sind Ausführungsformen vorstellbar, bei denen die Innenkontur 72 des Hohlschaftes 42 entlang ihrer Längserstreckung einen im Wesentlichen konstanten Querschnitt aufweist (D_{di} = Dₚᵢ). Es sind jedoch aus Ausgestaltungen vorstellbar, bei denen gilt D_{di} < Dₚᵢ. Gemäß diesen Ausführungsformen ist die Innenkontur 72 etwa kegelartig oder sphärisch gestaltet und weist in Richtung auf das distale Ende 48 eine Verjüngung auf.

Gemäß alternativer oder zusätzlicher Ausgestaltungen ist die Dicke der Wandung 70 entlang der Längserstreckung des Hohlschaftes 72 im Wesentlichen konstant (t_{d} = tₚ). Jedoch sind auch Ausgestaltungen denkbar, vergleiche insbesondere auch die in Fig. 3 gezeigte Ausführungsform, wobei sich die Wandstärke der Wandung 70 vom distalen Ende 48 hin zum proximalen Ende 50 erhöht (t_{d} < tₚ). Sämtliche der vorstehend genannten Ausführungsformen können miteinander kombiniert werden.

Wesentlich ist, dass die Innenkontur 72 derart an eine Außenkontur eines Trokardorns angepasst ist, dass sich in gewünschter Weise eine Aufweitung des Hohlschaftes 72 ergibt, wenn der Trokardorn in die Trokarhülse 40 eingeführt wird. Somit ist es von Vorteil, wenn zumindest der Hohlschaft 42 oder der Trokardorn zumindest abschnittsweise konisch, kegelig oder sphärisch gestaltet ist.

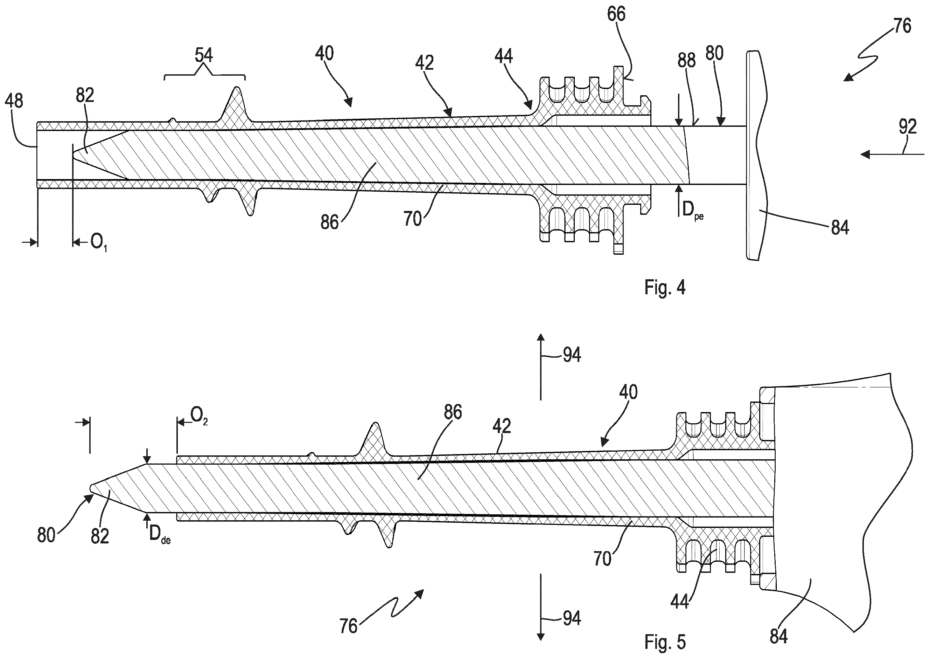

In diesem Zusammenhang wird ergänzend auf die Fig. 4, 5 und 6 Bezug genommen, die ein Trokarsystem 76 veranschaulichen, das eine Trokarhülse 40 etwa gemäß den Fig. 2 und 3 sowie einen mit 80 bezeichneten Trokardorn umfasst.

Wie bereits in Zusammenhang mit Fig. 1 veranschaulicht, weist der Trokardorn 80 an seinem distalen Ende eine Spitze 82 und an seinem proximalen Ende, das dem distalen Ende abgewandt ist, einen Handhabungsabschnitt 84 auf. Zwischen der Spitze 82 und dem Handhabungsabschnitt 84 erstreckt sich ein Schaft 86. Am Schaft 86 ist eine Umfangskontur oder Außenkontur 88 ausgebildet, die an der Innenkontur 72 des Hohlschaftes 42 der Trokarhülse 40 zur Anlage gelangt.

In Fig. 4 ist eine proximale Abmessung bzw. ein proximaler Durchmesser der Außenkontur 88 mit Dₚₑ beschrieben. In Fig. 5 ist eine distale Abmessung bzw. ein distaler Durchmesser der Außenkontur 88 mit D_{de} beschrieben. Gemäß zumindest einiger Ausgestaltungen gilt beim Trokardorn 80 Dₚₑ = D_{de}. Demgemäß ist der Schaft 86 des Trokardorns 80 zumindest entlang wesentlicher Abschnitte seiner Längserstreckung zylindrisch gestaltet und weist folglich einen konstanten Querschnitt auf. Es sind jedoch auch Gestaltungen vorstellbar, bei denen gilt D_{de} < Dₚₑ. Demgemäß kann auch der Schaft 86 des Trokardorns 80 in Richtung auf sein distales Ende zumindest abschnittsweise konisch oder sphärisch verjüngt sein. Es versteht sich, dass zusätzlich die Spitze 82 vorgesehen ist. Demgemäß ist es vorstellbar, dass zumindest die Innenkontur 72 oder die Außenkontur 88 in Richtung auf das distale Ende verjüngt gestaltet sind. Es ist jedoch auch vorstellbar, sowohl die Innenkontur 72 als auch die Außenkontur 88 zylinderförmig mit konstanten Querschnitten auszubilden. Ferner ist anzumerken, dass die Querschnitte der Innenkontur 72 und/oder der Außenkontur 88 nicht zwingend kreisförmig gestaltet sein müssen. Auch ovale oder in sonstiger Weise von einer reinen Kreisform abweichende Querschnitte sind vorstellbar. Insofern sind die hier verwendeten Bezeichnungen D_{di}, Dₚᵢ, D_{de} und Dₚₑ nicht zwingend als Durchmesserangaben zu verstehen.

Fig. 4 zeigt einen Zustand des Trokarsystems 76, bei dem der Trokardorn 80 in die Trokarhülse 40 eingeführt aber noch nicht vollständig durch die Hülse 40 durchgeführt ist, vergleiche ein Versatzmaß O₁ in Fig. 4 zwischen dem distalen Ende 48 der Trokarhülse 40 und der Spitze 82 des Trokardorns 80. Ferner ist in Fig. 4 eine Einführrichtung mit 92 bezeichnet.

In Fig. 5 ist der Trokardorn 80 vollständig in die Trokarhülse 40 eingeführt, so dass die Spitze 82 über das distale Ende 48 des Hohlschaftes 42 hinausragt, vergleiche ein in Fig. 5 mit O₂ bezeichnetes Versatzmaß. Ferner ist in Fig. 5 der Handhabungsabschnitt 84 des Trokardorns 80 zur Anlage an den Handhabungskopf 44 der Trokarhülse 40 gelangt. Der Handhabungsabschnitt 84 kontaktiert die proximale Abschlussfläche 66 des Handhabungskopfes 44.

Gemäß wesentlichen Aspekten der vorliegenden Offenbarung weitet der Trokardorn 80 den Hohlschaft 42 der Trokarhülse 40 auf, wenn der Trokardorn 80 in die Trokarhülse 40 eingeführt wird bzw. vollständig eingeführt ist. Ein entsprechendes radiales Ausrücken des Hohlschaftes 42 ist in Fig. 5 durch mit 94 bezeichnete Pfeile veranschaulicht. Mit anderen Worten weist die Außenkontur 88 des Trokardorns 80 gegenüber der Innenkontur 72 des Hohlschaftes 42 ein gewisses "Übermaß" auf, so dass der "weiche" Hohlschaft 42 aufgeweitet wird. Auf diese Weise werden beim Hohlschaft 42, insbesondere im Bereich der Innenkontur 72, der in unmittelbarem Kontakt mit der Außenkontur 88 ist, Spannungen erzeugt. Diese Spannungen bewirken eine Stabilisierung der eigentlich weichen, elastischen Trokarhülse 40. Der Hohlschaft 42 sitzt unter Vorspannung auf dem Trokardorn 80. Auf diese Weise wird beim Setzen des Trokars etwa ein Stauchen oder Strecken des Hohlschaftes 42 in Richtung von dessen Längserstreckung wirksam reduziert bzw. nahezu gänzlich unterbunden. Dasselbe gilt für eine Verdrillung (Tordierung) des Hohlschaftes 42 um seine Längsachse.

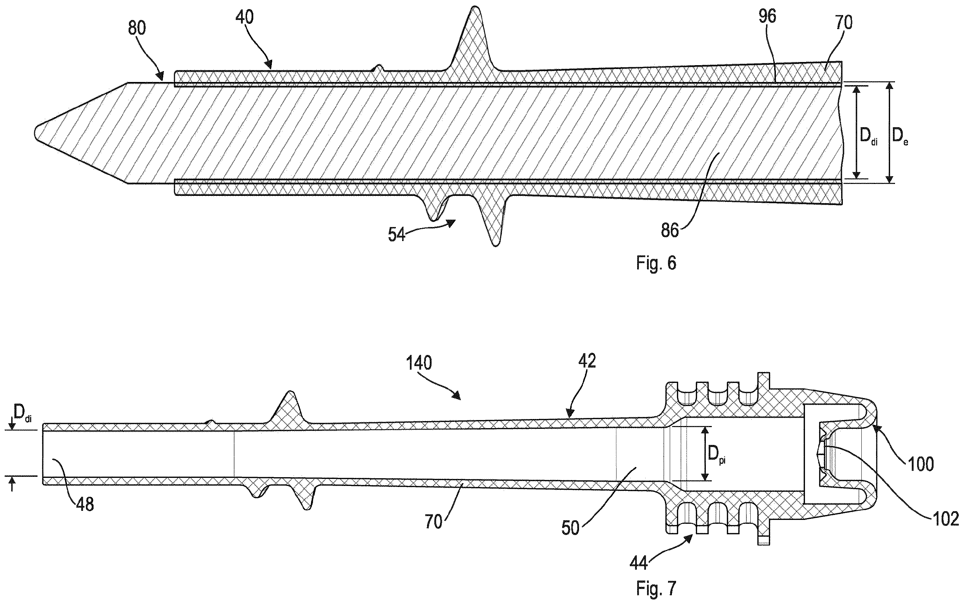

In Fig. 6 ist das "Übermaß" des Trokardorns 80 in Bezug auf die Innenkontur 72 des Hohlschaftes 42 in einem Überschneidungsbereich angedeutet, vergleiche die Außenabmessung (Außendurchmesser Dₑ) des Trokardorns 80 und die Innenabmessung (Innendurchmesser Tᵢ) des Hohlschaftes 42. Durch geeignete Auslegung der Innenkontur 72 und der Außenkontur 88 kann ein gewünschtes Übermaß definiert werden, welches zu einer definierten Vorspannung beim Hohlschaft 42 führt. Wie vorstehend bereits angedeutet, sind verschiedene Ausgestaltungen umfassend zylindrische, konische und/oder sphärische Gestaltungen denkbar, um eine gewünschte Spannungscharakteristik herbeizuführen. Dies kann insbesondere ein Verhältnis zwischen der erzielten Vorspannung beim Hohlschaft 72 und der aktuellen axialen Position des Trokardorns 80 in Bezug auf die Trokarhülse 40 betreffen.

Fig. 7 veranschaulicht eine abgewandelte Ausführungsform einer Trokarhülse, die mit 140 bezeichnet ist. Die Trokarhülse 140 ist grundsätzlich der anhand der Fig. 2 bis 6 veranschaulichten Trokarhülse 40 ähnlich gestaltet. Die Trokarhülse 140 ist in vorstehend bereits beschriebener Weise mit einem Hohlschaft 42 und einem Handhabungskopf 44 versehen. An einem proximalen Ende des Handhabungskopfes 44 ist eine Dichtung 100 ausgebildet, die eine umlaufende Dichtungskontur bzw. Dichtlippe 102 aufweist. Der Schraffur des Längsschnitts durch die Trokarhülse 140 gemäß Fig. 7 kann entnommen werden, dass die Dichtung 100 als integraler Bestandteil der Trokarhülse 140 ausgebildet ist. Da die Trokarhülse 140 integral aus einem elastomeren Werkstoff gefertigt ist, können auch Dichtungsanordnungen integriert und einstückig mit dem Hohlschaft 42 und dem Handhabungskopf 44 ausgebildet werden.

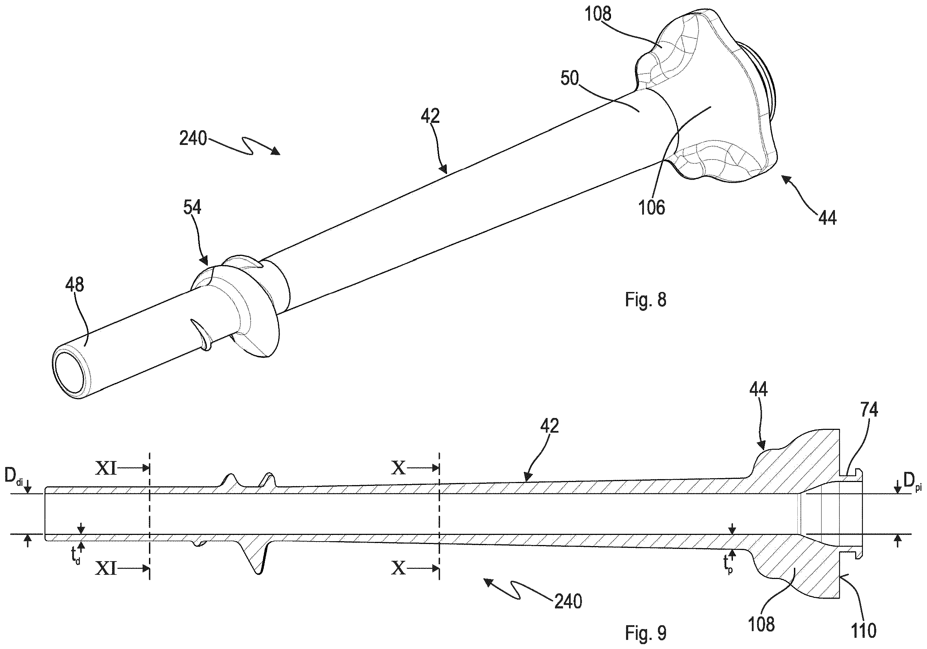

Mit Bezugnahme auf die Fig. 8 und 9 wird eine weitere Ausführungsform einer Trokarhülse veranschaulicht, die mit 240 bezeichnet wird. Auch die Trokarhülse 240 ist grundsätzlich der Trokarhülse 40 ähnlich gestaltet und mit einem Hohlschaft 42 sowie einem Handhabungskopf 44 versehen. Ferner ist beispielhaft ein Gewindeabschnitt 54 ausgebildet.

Der in Fig. 9 gezeigten Schnittdarstellung kann entnommen werden, dass eine Stärke oder Dicke der Wandung 70 des Hohlschaftes 42 vom distalen Ende 48 hin zum proximalen Ende 50 deutlich zunimmt, vergleiche die Abmessungen t_{d} sowie tₚ. Somit kann ein gewünschter Festigkeitsverlauf bzw. Vorspannungsverlauf entlang der Längsachse des Hohlschaftes 42 bewirkt werden, insbesondere wenn ein Trokardorn in die Trokarhülse 240 eingeführt ist.

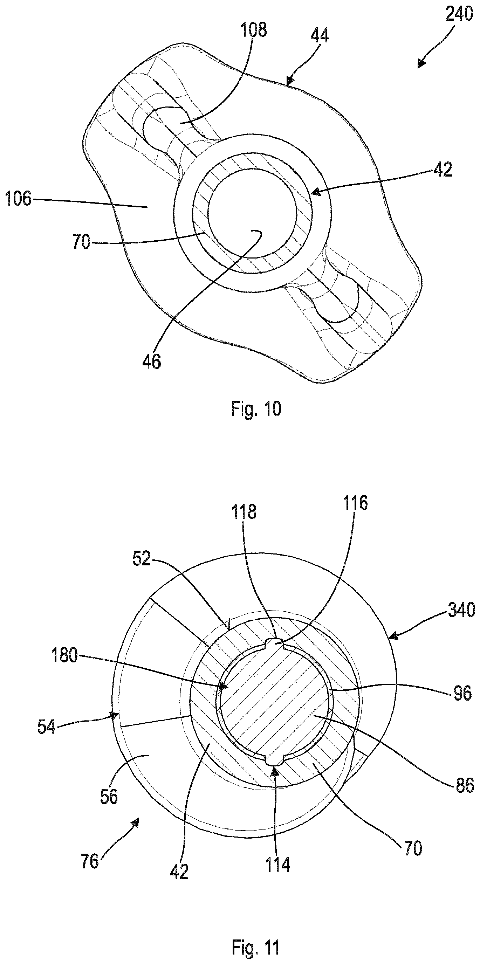

In Abwandlung gegenüber den vorstehend veranschaulichten Ausführungsbeispielen ist der Handhabungskopf 44 in Fig. 8 und Fig. 9 kompakt gestaltet und nicht mit Versteifungen versehen. Auch eine derartige Gestaltung ist denkbar. Am Handhabungskopf 44 ist eine Aufnahmekontur bzw. Aufnahme 74 für eine Dichtung ausgebildet, vergleiche Fig. 9. Ausgehend vom Hohlschaft 42 weist der Handhabungskopf 44 einen konkaven Übergang 106 auf. Ferner sind rippenartige Erhebungen 108 ausgebildet, vergleiche hierzu auch Fig. 10, die nachfolgend näher erläutert wird. Das in den Fig. 8 und 9 gezeigte Ausführungsbeispiel umfasst zwei um 180° zueinander versetzt angeordnete rippenartige Erhebungen 108, die gegenüber dem konkaven Übergang 106 erhaben ausgeformt sind. Der Handhabungskopf 44 mündet in eine proximale Abschlussfläche 110, der jedoch die Aufnahme 74 proximal vorgelagert sein kann.

Fig. 10 veranschaulicht einen axialen Schnitt durch die anhand der Fig. 8 und 9 veranschaulichte Trokarhülse 240. In Fig. 9 ist ein Schnittverlauf durch eine Linie X-X angedeutet. Durch das Einführen des Trokardorns kann die Wandung 70 des Hohlschaftes 42 aufgeweitet werden. In Fig. 10 umschließt die Wandung 70 einen kreisförmigen Durchlass 46. Es versteht sich, dass auch andere Profile vorstellbar sind.

In diesem Zusammenhang wird ergänzend auf Fig. 11 verwiesen, die einen weiteren Axialschnitt durch eine Trokarhülse veranschaulicht, die mit 340 bezeichnet ist. In der Trokarhülse 340 ist ein Trokardorn 180 aufgenommen. Die anhand der Fig. 11 veranschaulichte Ausgestaltung der Trokarhülse 340 entspricht nicht vollständig der Ausgestaltung der Trokarhülse 240 gemäß Fig. 9. Gleichwohl ist vorrangig zu Veranschaulichungszwecken in Fig. 9 ein Schnittverlauf durch eine mit XI-XI bezeichnete Linie angedeutet.

Fig. 11 zeigt einen Blick vom proximalen Ende her auf den Gewindegang 56 des Gewindeabschnitts 54. Wie vorstehend bereits erläutert ist eine Höhe des Gewindegangs 56 in Bezug auf eine Oberfläche 52 des Hohlschaftes 42 variabel.

Die Trokarhülse 340 sowie der Trokardorn 180 gemäß der Ausgestaltung in Fig. 11 unterscheiden sich ferner von den vorstehend beschriebenen Ausführungsformen durch eine Verdrehsicherung 114. Die Verdrehsicherung 114 umfasst beim Trokardorn 180 zumindest ein Lagesicherungselement 116. Die Verdrehsicherung 114 umfasst bei der Trokarhülse 340 zumindest ein Lagesicherungselement 118. Bei dem Lagesicherungselement 116 handelt es sich um eine sich in Längsrichtung erstreckende Erhebung. Bei dem Lagesicherungselement 118 handelt es sich um eine sich in Längsrichtung erstreckende Vertiefung, die an die Erhebung angepasst ist. In Fig. 11 umfasst die Verdrehsicherung 114 jeweils zwei um 180° versetzte Paare von Lagesicherungselementen 116, 118.

Sofern beispielsweise durch äußere Kräfte entweder die Trokarhülse 340 oder der Trokardorn 180 gegenüber dem jeweils anderen Bauteil verdreht wird, kommt es insbesondere im Bereich der Lagesicherungselemente 116, 118 zu einer Erhöhung der Vorspannung im Hohlschaft 42, wenn die Lagesicherungselement 116, 118 ausrücken wollen. Dies führt zu einer deutlichen lokalen Aufweitung und somit zu einer weiteren Erhöhung der Eigenstabilität des Hohlschaftes 42 der Trokarhülse 340. Mit anderen Worten wird die Stabilität des Hohlschaftes 42 umso mehr erhöht, umso größer das eigentlich deformierende Moment ist.

Es versteht sich, dass die Verdrehsicherung 114 auch andere Ausführungen von Lagesicherungselementen 116, 118 umfassen kann. Beispielhaft können Profile der Innenkontur 72 und der Außenkontur 88 oval oder in anderer Weise nichtkreisförmig gestaltet sein.

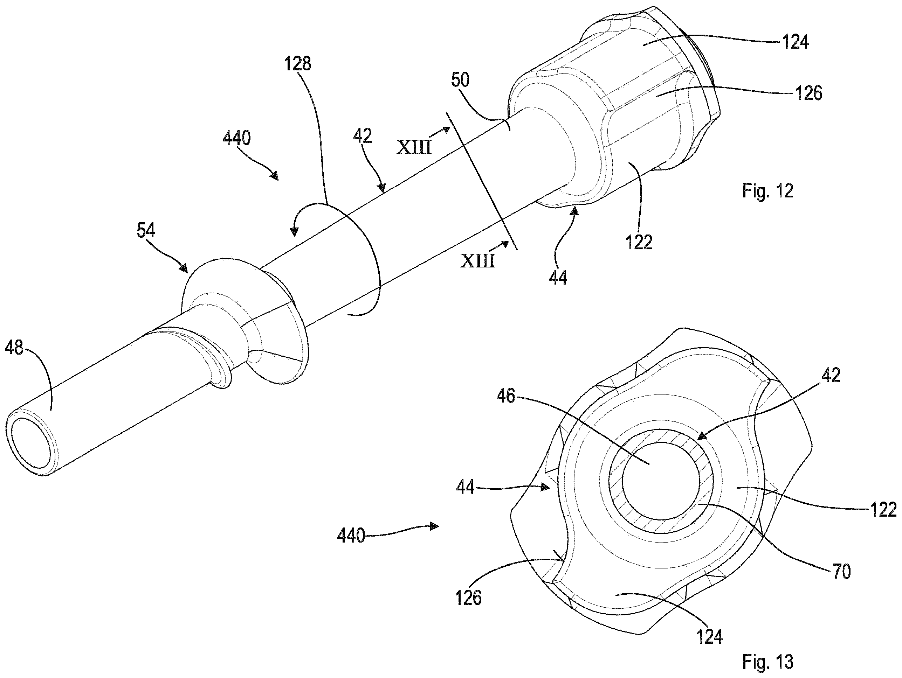

Mit ergänzendem Bezug auf die Fig. 12 und 13 wird eine weitere Ausführungsform einer Trokarhülse veranschaulicht, die mit 440 bezeichnet ist. Die Trokarhülse 440 entspricht vom Grundsatz her der Ausgestaltung der anhand der Fig. 2 bis 6 veranschaulichten Trokarhülse 40.

Fig. 13 veranschaulicht einen axialen Schnitt durch die Trokarhülse 440, der aus Veranschaulichungsgründen in der perspektivischen Darstellung von Fig. 12 durch eine Linie XIII-XIII dargestellt ist.

In erster Linie unterscheidet sich die Trokarhülse 440 von den Ausgestaltungen gemäß den Fig. 2 bis 6 durch die Gestaltung des Handhabungskopfes 44. Der Handhabungskopf 44 der Trokarhülse 440 ist kompakt gestaltet und weist keine Rippen und Vertiefungen auf. Insofern weist der Handhabungskopf 44 zwar Materialanhäufungen auf, dies ist jedoch bei den gewählten Werkstoffen bzw. Herstellverfahren nicht sonderlich nachteilig. Der frontalen Ansicht von Fig. 13 kann entnommen werden, dass der Handhabungskopf 44 eine Aufdickung 122 umfasst, wobei sich Erhebungen 124 ausgehend von der Aufdickung 122 radial nach außen erstrecken. Beispielhaft sind zwei um 180° versetzte Erhebungen 124 vorgesehen. Die Erhebungen 124 umfassen jeweils eine Mitnahmeflanke 126, über die eine Kraft eingeleitet werden kann, etwa um die Trokarhülse 440 zu verdrehen, vergleiche einen in Fig. 12 mit 128 bezeichneten Pfeil zur Veranschaulichung der Drehrichtung. Auf diese Weise kann die Trokarhülse 440, idealerweise ein Trokarsystem umfassend die Trokarhülse 440 sowie einen darin aufgenommenen Trokardorn, betätigt werden, um etwa den Gewindeabschnitt 54 einzuschrauben. Auf diese Weise wird das Einführen des Trokarsystems vereinfacht.

Im Hinblick auf die Erhebungen 124, die mit entsprechenden Mitnahmeflanken 126 versehen sind, wird angemerkt, dass auch die Trokarhülse 40 in Fig. 2 eine zumindest ähnliche Gestaltung aufweist, wobei dort eben die Aufdickung (122 in Fig. 12) durch Rippen 58 und Ausnehmungen 62 ersetzt ist.

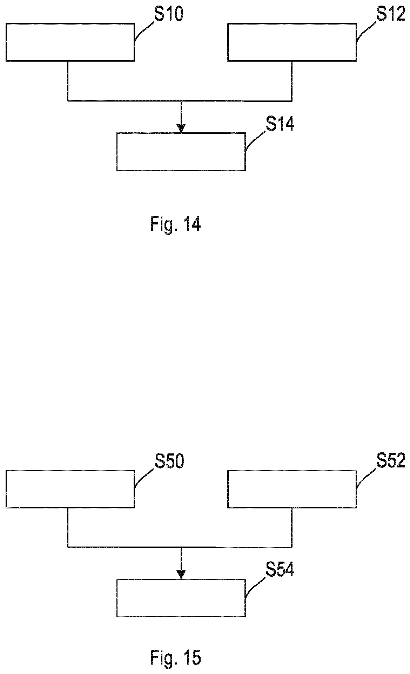

Mit Bezugnahme auf Fig. 14 wird anhand eines Blockdiagramms eine beispielhafte Ausgestaltung eines Verfahrens zur Herstellung einer Trokarhülse veranschaulicht. Das Verfahren umfasst einen Schritt S10, der die Bereitstellung einer Form beinhaltet, die zur Erzeugung einer Trokarhülse mit einem flexiblen Hohlschaft und einem Handhabungskopf ausgebildet ist. Die Form umfasst üblicherweise zumindest eine Kavität, die gewissermaßen ein Negativ der gewünschten Trokarhülse darstellt. In einem weiteren Schritt S12 wird ein elastomerer Werkstoff bereitgestellt, vorzugsweise ein Werkstoff, der im verarbeiteten Zustand eine definierte Shore Härte aufweist. Eine hinreichende Flexibilität ist bei einem Werkstoff gegeben, dessen Shore Härte, bestimmt gemäß DIN ISO 7619-1, maximal 90 Shore A, vorzugsweise maximal 70 Shore A, weiter bevorzugt maximal 50 Shore A beträgt. Gemäß einer beispielhaften Ausführungsform beträgt die Shore Härte 70 Shore A.

An die Schritte S10 und S12 schließt sich ein weiterer Schritt S14 an, der die urformende Fertigung der Trokarhülse betrifft. Der Schritt S14 umfasst ein Befüllen der Form mit einem geeigneten Ausgangswerkstoff. Das Urformen umfasst ferner ein einstückiges Ausbilden des Hohlschaftes und des Handhabungskopfes. Am Hohlschaft ist hierfür eine Innenkontur ausgewählt, die derart an einer Außenkontur eines Trokardorns angepasst ist, dass die Trokarhülse und der Trokardorn unter Erzeugung einer definierten Vorspannung miteinander koppelbar sind, um die Trokarhülse definiert zu führen.

Mit Bezugnahme auf Fig. 15 wird anhand eines Blockdiagramms ein Verfahren zur Bereitstellung eines Trokarsystems veranschaulicht. Das Verfahren umfasst einen Schritt S50, der die Herstellung einer Trokarhülse umfasst, insbesondere gemäß dem anhand der Fig. 14 veranschaulichten Herstellungsverfahren. Das Verfahren umfasst einen weiteren Schritt S52, der die Bereitstellung eines Trokardorns umfasst, der an die Trokarhülse angepasst ist. Insbesondere weist der Trokardorn eine Außenkontur auf, die an eine Innenkontur des Hohlschaftes der Trokarhülse angepasst ist. Dies umfasst insbesondere Ausgestaltungen, bei denen die Außenkontur des Trokardorns bewusst etwas größer als die korrespondierende Innenkontur des Hohlschaftes gewählt ist.

Schließlich folgt ein Schritt S54, der ein Einführen des Trokardorns in die Trokarhülse betrifft, wobei die Trokarhülse zumindest abschnittsweise aufgeweitet wird. Dabei wird eine definierte Vorspannung im Hohlschaft der Trokarhülse erzeugt. Dies kann insbesondere zur mechanischen Stabilisierung der Trokarhülse im Verbund mit dem Trokardorn genutzt werden.

## Patentansprüche

1. Trokarsystem (76), mit einer Trokarhülse (40) und einem Trokardorn (80), wobei die Trokarhülse (40) mit einem flexiblen Hohlschaft (42), der ein distales Ende und ein proximales Ende aufweist, und mit einem Handhabungskopf (44) versehen ist, der am proximalen Ende des Hohlschaftes (42) ausgebildet ist, und wobei die Trokarhülse (40) aus einem elastomeren Werkstoff hergestellt ist, **dadurch gekennzeichnet, dass** der Hohlschaft (42) eine Innenkontur (72) aufweist, die derart an eine Außenkontur (88) des Trokardorns (80) angepasst ist, dass die Trokarhülse (40) und der Trokardorn (80) unter Erzeugung einer definierten Vorspannung miteinander koppelbar sind, um die Trokarhülse (40) zu stabilisieren, dass der Trokardorn (80) unter Erzeugung der definierten Vorspannung in die Trokarhülse (40) einführbar ist, und dass zumindest der Hohlschaft (42) und der Handhabungskopf (44) einstückig gestaltet sind.

2. Trokarsystem (76) nach Anspruch 1, **dadurch gekennzeichnet, dass** der Hohlschaft (42) in einer definierten axialen Relativlage zwischen der Trokarhülse (40) und dem Trokardorn (80) zumindest abschnittsweise aufgeweitet ist.

3. Trokarsystem (76) nach Anspruch 1 oder 2, **dadurch gekennzeichnet, dass** die Shore Härte des elastomeren Werkstoffs, bestimmt gemäß DIN ISO 7619-1, maximal 90 Shore A, vorzugsweise maximal 70 Shore A oder etwa 70 Shore A, beträgt.

4. Trokarsystem (76) nach einem der vorhergehenden Ansprüche, **dadurch gekennzeichnet, dass** die Trokarhülse (40) integral gestaltet und urformend hergestellt ist.

5. Trokarsystem (76) nach einem der vorhergehenden Ansprüche, **dadurch gekennzeichnet, dass** der elastomere Werkstoff aus der Gruppe ausgewählt ist, die aus Folgendem besteht: Silikone, Silikonkautschuk, thermoplastische Elastomere, Kautschuk sowie diese enthaltende Compounds.

6. Trokarsystem (76) nach einem der vorhergehenden Ansprüche, **dadurch gekennzeichnet, dass** der Hohlschaft (42) zumindest abschnittweise rohrartig oder zylinderartig gestaltet ist.

7. Trokarsystem (76) nach Anspruch 6, **dadurch gekennzeichnet, dass** der Hohlschaft (42) zumindest abschnittweise eine glatte, erhebungsfreie Außenfläche aufweist.

8. Trokarsystem (76) nach Anspruch 6 oder 7, **dadurch gekennzeichnet, dass** die Innenkontur (72) zumindest abschnittsweise mit einer reibungsmindernden Beschichtung versehen ist.

9. Trokarsystem (76) nach einem der vorhergehenden Ansprüche, **dadurch gekennzeichnet, dass** der Hohlschaft (42) zumindest abschnittsweise eine sich von einem distalen Ende hin zu einem proximalen Ende erhöhende Wandstärke aufweist.

10. Trokarsystem (76) nach einem der vorhergehenden Ansprüche, **dadurch gekennzeichnet, dass** die Innenkontur (72) des Hohlschaftes (42) zumindest abschnittsweise konisch oder sphärisch mit einem von einem distalen Ende hin zu einem proximalen Ende größer werdenden Innendurchmesser gestaltet ist.

11. Trokarsystem (76) nach Anspruch 10, **dadurch gekennzeichnet, dass** die Innenkontur (72) des Hohlschaftes (42) an eine zumindest abschnittsweise konische oder sphärische Gestaltung der Außenkontur (88) des Trokardorns (80) angepasst ist, um die Trokarhülse (40) beim Einführen des Trokardorns (80) definiert vorzuspannen.

12. Trokarsystem (76) nach einem der vorhergehenden Ansprüche, **dadurch gekennzeichnet, dass** am Hohlschaft (42) ein Gewindeabschnitt (54) ausgebildet ist, und wobei der Hohlschaft (42), der Gewindeabschnitt (54) und der Handhabungskopf (44) einstückig gestaltet sind.

13. Trokarsystem (76) nach einem der vorhergehenden Ansprüche, **dadurch gekennzeichnet, dass** die Innenkontur (72) des Hohlschaftes (42) zumindest abschnittsweise nicht-rotationssymmetrisch ausgeführt ist und insbesondere einen unrunden Querschnitt aufweist.

14. Trokarsystem (76) nach einem der vorhergehenden Ansprüche, **dadurch gekennzeichnet, dass** am proximalen Ende des Hohlschaftes (42), insbesondere beim Handhabungskopf (44), eine Dichtungskontur (100) oder eine Dichtungsaufnahmekontur (74) ausgebildet ist.

15. Verfahren zur Bereitstellung eines Trokarsystems (76), umfassend:
- Herstellung einer Trokarhülse (40), aufweisend die folgenden Schritte:
- Bereitstellung einer Form, die zur Erzeugung einer Trokarhülse (40) mit einem flexiblen Hohlschaft (42) und einem Handhabungskopf (44) ausgebildet ist,
- Bereitstellung eines elastomeren Werkstoffs, vorzugsweise eines Werkstoffs der im verarbeiteten Zustand eine Shore Härte, bestimmt gemäß DIN ISO 7619-1, von maximal 90 Shore A, vorzugsweise maximal 70 Shore A, aufweist, und
- Urformen der Trokarhülse (40), umfassend Befüllen der Form, dabei einstückiges Formen des Hohlschaftes (42) und des Handhabungskopfes (44), wobei am Hohlschaft (42) eine Innenkontur (72) ausgebildet wird, die derart an eine Außenkontur (88) eines Trokardorns (80) angepasst ist, dass die Trokarhülse (40) und der Trokardorn (80) unter Erzeugung einer definierten Vorspannung miteinander koppelbar sind, um die Trokarhülse (40) zu stabilisieren,
- Bereitstellung eines Trokardorns (80), der an die Trokarhülse (40) angepasst ist, und
- Einführen des Trokardorns (80) in die Trokarhülse (40), dabei zumindest abschnittsweises Aufweiten der Trokarhülse (40), umfassend Erzeugung einer Vorspannung im Hohlschaft (42) der Trokarhülse (40) zur mechanischen Stabilisierung der Trokarhülse (40).

## Claims

1. Trocar system (76), having a trocar sleeve (40) and a trocar mandrel (80), wherein the trocar sleeve (40) comprising a flexible hollow shaft (42) comprising a distal end and a proximal end, and a handling head (44) that is formed at the proximal end of the hollow shaft (42), and wherein the trocar sleeve (40) is manufactured from an elastomer material, **characterized in that** the hollow shaft (42) comprises an inner contour (72) that is adapted to an outer contour (88) of the trocar mandrel (80) such that the trocar sleeve (40) and the trocar mandrel (80) are arranged to be coupled to one another while generating a defined preloading to stabilize the trocar sleeve (40), that the trocar mandrel (80) is insertable in the trocar sleeve (40) while generating the defined preloading, and that at least the hollow shaft (42) and the handling head (44) are formed in one piece.

2. Trocar system (76) according to claim 1, **characterized in that** the hollow shaft (42) is, in a defined axial relative position between the trocar sleeve (40) and the trocar mandrel (80), at least sectionally widened.

3. Trocar system (76) according to claim 1 or 2, **characterized in that** the elastomer material has a Shore hardness, determined in accordance with DIN ISO 7619-1, of no more than 90 Shore A, preferably of no more than 70 Shore A.

4. Trocar system (76) according to any of the preceding claims, **characterized in that** the trocar sleeve (40) is integrally shaped and manufactured by casting.

5. Trocar system (76) according to any of the preceding claims, **characterized in that** the elastomer material is selected from the group consisting of the following:
silicone, silicone rubber, thermoplastic elastomers, rubber, and compounds containing the same.

6. Trocar system (76) according to any of the preceding claims, **characterized in that** the hollow shaft (42) is at least sectionally tubular or cylindrically shaped

7. Trocar system (76) according to claim 6, **characterized in that** the hollow shaft (42) comprises at least sectionally a smooth, elevation-free outer surface.

8. Trocar system (76) according to claim 6 or 7, **characterized in that** the inner contour (72) is at least sectionally provided with a friction-reducing coating.

9. Trocar system (76) according to any of the preceding claims, **characterized in that** the hollow shaft (42) at least sectionally comprises a wall thickness that is increasing from a distal end towards a proximal end.

10. Trocar system (76) according to any of the preceding claims, **characterized in that** the inner contour (72) of the hollow shaft (42) is at least sectionally tapered or spherically shaped, having an inner diameter that is increasing from a distal end towards a proximal end.

11. Trocar system (76) according to claim 10, **characterized in that** the inner contour (72) of the hollow shaft (42) is adapted to an at least sectionally tapered or spherical shape of the outer contour (88) of the trocar mandrel (80) to preload the trocar sleeve (40) in a defined fashion when the trocar mandrel (80) is inserted.

12. Trocar system (76) according to any of the preceding claims, **characterized in that** a thread section (54) is formed at the hollow shaft (42), wherein the hollow shaft (42), the thread section (54) and the handling head (44) are formed in one piece.

13. Trocar system (76) according to any of the preceding claims, **characterized in that** the inner contour (72) of the hollow shaft (42) is at least sectionally non-rotationally symmetric and particularly provided with a non-circular cross-section.

14. Trocar system (76) according to any of the preceding claims, **characterized in that** a sealing contour (100) or a sealing receiving contour (74) is formed at the proximal end of the hollow shaft (42), particularly at the handling head (44).

15. Method for providing a trocar system (76), comprising:
- manufacturing a trocar sleeve (40), comprising the following steps:
- providing a mold that is configured for forming a trocar sleeve (40) having a flexible hollow shaft (42) and a handling head (44),
- providing an elastomer material, particularly a material having, in the processed state, a Shore hardness, determined in accordance with DIN ISO 7619-1, of no more than 90 Shore A, preferably of no more than 70 Shore A,
- casting the trocar sleeve (40), involving filling the mold, including integrally forming the hollow shaft (42) and the handling head (44), wherein at the hollow shaft (42) an inner contour (72) is formed that is adapted to an outer contour (88) of a trocar mandrel (80) in such a way that the trocar sleeve (40) and the trocar mandrel (80) are arranged to be coupled to one another while generating a defined preload to stabilize the trocar sleeve (40),
- providing a trocar mandrel (80) that is adapted to the trocar sleeve (40), and
- inserting the trocar mandrel (80) in the trocar sleeve (40), including at least sectionally widening the trocar sleeve (40), involving generating a preloading, in the hollow shaft (42) of the trocar sleeve (40), for mechanically stabilizing the trocar sleeve (40).

## Revendications

1. Système de trocart (76), comportant une chemise de trocart (40) et un mandrin de trocart (80), la chemise de trocart (40) étant dotée d'une tige creuse flexible (42), qui présente une extrémité distale et une extrémité proximale, et d'une tête de manipulation (44) qui est réalisée à l'extrémité proximale de la tige creuse (42), et la chemise de trocart (40) étant fabriquée à partir d'une matière élastomère, **caractérisé en ce que** la tige creuse (42) présente un contour intérieur (72) qui est adapté au contour extérieur (88) du mandrin de trocart (80) de telle sorte que la chemise de trocart (40) et le mandrin de trocart (80) puissent être accouplés l'un à l'autre en produisant une précontrainte définie, afin de stabiliser la chemise de trocart (40), **en ce que** le mandrin de trocart (80) peut être inséré dans la chemise de trocart (40) en produisant la précontrainte définie, et **en ce qu'**au moins la tige creuse (42) et la tête de manipulation (44) sont configurées d'une seule pièce.

2. Système de trocart (76) selon la revendication 1, **caractérisé en ce que** la tige creuse (42) est élargie au moins dans certaines parties dans une position relative axiale définie entre la chemise de trocart (40) et le mandrin de trocart (80).

3. Système de trocart (76) selon la revendication 1 ou 2, **caractérisé en ce que** la dureté Shore de la matière élastomère, déterminée selon DIN ISO 7619-1, est d'au maximum 90 Shore A, de préférence d'au maximum 70 Shore A ou d'approximativement 70 Shore A.

4. Système de trocart (76) selon l'une des revendications précédentes, **caractérisé en ce que** la chemise de trocart (40) est configurée intégralement et fabriquée par façonnage.

5. Système de trocart (76) selon l'une des revendications précédentes, **caractérisé en ce que** la matière élastomère est sélectionnée dans le groupe qui est constitué des éléments suivants : silicone, caoutchouc au silicone, élastomères thermoplastiques, caoutchouc ainsi que composés contenant ceux-ci.

6. Système de trocart (76) selon l'une des revendications précédentes, **caractérisé en ce que** la tige creuse (42) est configurée de manière tubulaire ou cylindrique au moins dans certaines parties.

7. Système de trocart (76) selon la revendication 6, **caractérisé en ce que** la tige creuse (42) présente au moins dans certaines parties une surface extérieure lisse sans rehaussements.

8. Système de trocart (76) selon la revendication 6 ou 7, **caractérisé en ce que** le contour intérieur (72) est doté, au moins dans certaines parties, d'un revêtement réduisant la friction.

9. Système de trocart (76) selon l'une des revendications précédentes, **caractérisé en ce que** la tige creuse (42) présente, au moins dans certaines parties, une épaisseur de paroi augmentant à partir d'une extrémité distale vers une extrémité proximale.

10. Système de trocart (76) selon l'une des revendications précédentes, **caractérisé en ce que** le contour intérieur (72) de la tige creuse (42) est configuré au moins dans certaines parties de manière conique ou sphérique avec un diamètre intérieur croissant à partir d'une extrémité distale vers une extrémité proximale.

11. Système de trocart (76) selon la revendication 10, **caractérisé en ce que** le contour intérieur (72) de la tige creuse (42) est adapté à une configuration, conique ou sphérique au moins dans certaines parties, du contour extérieur (88) du mandrin de trocart (80), afin de précontraindre de manière définie la chemise de trocart (40) lors de l'insertion du mandrin de trocart (80).

12. Système de trocart (76) selon l'une des revendications précédentes, **caractérisé en ce qu'**une partie filetée (54) est réalisée sur la tige creuse (42), et la tige creuse (42), la partie filetée (54) et la tête de manipulation (44) étant configurées d'une seule pièce.

13. Système de trocart (76) selon l'une des revendications précédentes, **caractérisé en ce que** le contour intérieur (72) de la tige creuse (42) est réalisé de manière à ne pas présenter de symétrie de révolution au moins dans certaines parties et présente en particulier une section transversale non circulaire.

14. Système de trocart (76) selon l'une des revendications précédentes, **caractérisé en ce qu'**un contour de joint d'étanchéité (100) ou un contour de logement de joint d'étanchéité (74) est réalisé à l'extrémité proximale de la tige creuse (42), en particulier près de la tête de manipulation (44).

15. Procédé de fourniture d'un système de trocart (76), comportant :
- la fabrication d'une chemise de trocart (40), présentant les étapes suivantes :
- fourniture d'un moule qui est réalisé pour la production d'une chemise de trocart (40) dotée d'une tige creuse flexible (42) et d'une tête de manipulation (44),
- fourniture d'une matière élastomère, de préférence d'une matière qui présente à l'état traité une dureté Shore, déterminée selon DIN ISO 7619-1, d'au maximum 90 Shore A, de préférence d'au maximum 70 Shore A, et
- façonnage de la chemise de trocart (40), comportant le remplissage du moule, en l'occurrence moulage d'une seule pièce de la tige creuse (42) et de la tête de manipulation (44), un contour intérieur (72) étant réalisé sur la tige creuse (42), lequel est adapté à un contour extérieur (88) d'un mandrin de trocart (80) de telle sorte que la chemise de trocart (40) et le mandrin de trocart (80) puissent être accouplés l'un à l'autre en produisant une précontrainte définie, afin de stabiliser la chemise de trocart (40),
- fourniture d'un mandrin de trocart (80) qui est adapté à la chemise de trocart (40), et
- insertion du mandrin de trocart (80) dans la chemise de trocart (40), en l'occurrence élargissement, au moins dans certaines parties, de la chemise de trocart (40), comportant la production d'une précontrainte dans la tige creuse (42) de la chemise de trocart (40) pour la stabilisation mécanique de la chemise de trocart (40).
